# FASCICULE DE BREVET EUROPEEN

(11) **EP 0 855 907 B1**
(45) Date de publication et mention de la délivrance du brevet: **24.07.2002**
(21) Numéro de dépôt: 96934932.3
(22) Date de dépôt: 17.10.1996
(51) Int. Cl.: A61K 9/70, A61K 9/12

(54) **COMPOSITION POUR L'ADMINISTRATION TRANSDERMIQUE**
ZUSAMMENSETZUNG ZUR TRANSDERMALEN WIRKSTOFFABGABE
COMPOSITION FOR TRANSDERMAL DELIVERY

(30) Priorité: 20.10.1995 FR 9512393
(43) Date de publication de la demande: 05.08.1998
(73) Titulaire: LABORATOIRE L. LAFON, 94701 Maisons Alfort (FR)
(72) Inventeur: LAURENT, Philippe, F-69600 Oulins (FR)
(74) Mandataire: Bernasconi, Jean Raymond
(86) Numéro de dépôt international: FR9601628
(87) Numéro de publication internationale: WO9715295

(56) Documents cités:
- EP-A- 0 478 456
- EP-A- 0 679 392
- WO-A-95/30409
- US-A- 3 836 647
- PATENT ABSTRACTS OF JAPAN vol. 13, no. 556 (C-664), 11 Décembre 1989 & JP 01 230514 A (OSAKA AEROSOL IND CORP), 14 Septembre 1989,
- PATENT ABSTRACTS OF JAPAN vol. 13, no. 278 (C-611), 26 Juin 1989 & JP 01 075416 A (SHISEIDO CO LTD), 22 Mars 1989,

## Description

La présente invention concerne une nouvelle composition pour l'administration transdermique d'un principe actif.

On a développé dans les années 1980 des systèmes transdermiques qui sont appliqués sur une surface délimitée de la peau et qui servent de support ou de véhicule à un ou plusieurs principes actifs, destinés généralement à exercer une action générale après libération et passage à travers la barrière cutanée.

Ces systèmes, appelés généralement "patchs transdermiques", présentent par rapport aux formes dermatologiques classiques telles que onguents, pommades, gels, solutions, lotions, un certain nombre d'avantages:
- passage direct et continu dans la circulation générale,
- suppression du premier passage hépatique et/ou des dégradations dans le tractus digestif avec pour conséquence une diminution des effets secondaires,
- prolongation de la durée d'action,
- maintien d'un taux constant de principes actifs dans le plasma,
- augmentation de l'observance par diminution de la fréquence des prises,
- diminution des variations inter-individuelles,
- maîtrise de la dose administrée grâce à un système matriciel ou membranaire à réservoir,
- obtention d'une concentration constante de principe actif durant la durée de l'application.
   Malgré le degré d'innovation apporté par ces systèmes, il n'existe aujourd'hui que très peu de spécialités sous cette forme. Ceci est dû au fait que ces dispositifs exigent :
   . une technologie de fabrication très sophistiquée,
   . des sites de production rares qui appartiennent à quelques grands groupes qui en ont le monopole,
   . ceci entraîne un coût élevé de fabrication et un prix de revient et de vente important. Ces systèmes sont en définitif réservés à des produits chers;

EP-A-0 478 456 décrit des compositions antifongiques sous forme de spray contenant un polymère siliconé.

La présente invention vise à fournir de nouvelles compositions pour l'administration transdermique d'un principe actif
- qui soient très simples à mettre en oeuvre, ne demandent pas d'installations industrielles lourdes, compliquées et onéreuses,
- qui soient intéressantes sur le plan économique avec un moindre coût de réalisation.

A cet effet, la présente invention a pour objet une. composition destinée à former sur la peau, par pulvérisation à partir d'un boîtier pour aérosol, une pellicule pour l'administration transdermique d'un principe actif, qui comprend :
a) un principe actif lipophile
b) de 0,5 à 25% en poids, et avantageusement de 5 à 25% en poids, d'une composition polymère adhésive à base de silicone
c) de 0 à 25% en poids d'un promoteur d'absorption
d) de 25 à 95% en poids d'un solvant volatil comprenant des silicones volatiles, et
e) de 0,5 à 50% en poids d'un gaz propulseur sous pression

Dans la présente invention, par principe actif on désigne principalement un médicament ou une substance ayant des propriétés thérapeutiques.

Ces médicaments sont notamment des vitamines lipophiles telles que les vitamines D et E et leurs dérivés, des hormones telles que la calcitonine, des stéroïdes tels que l'estradiol et ses esters et la prednisone, la nicotine, des corticoïdes, des dérivés rétinoïques, des antimycosiques tels que le kétoconazole, des anesthésiques, des antalgiques tels que la lidocaïne ou des anticancéreux cutanés.

Les pourcentages des principes actifs dans les compositions de l'invention dépendent évidemment de la nature du principe actif. Généralement les pourcentages sont de 0,01 à 10% en poids.

Selon l'invention, on entend par composition polymère à base de silicone une composition contenant aussi bien des polymères à base de silicones que des copolymères à base de silicones.

Ces silicones, qui seront désignés selon la nomenclature du dictionnaire de la CTFA (Cosmetic, Toiletry and Fragrance Association), comprennent notamment des huiles polydiméthylsiloxanes ou des huiles polydiméthylsiloxanes modifiées par des groupes organiques ioniques ou non ioniques.

Comme exemple d'huiles polydiméthylsiloxanes, on citera les diméthicones de formule : 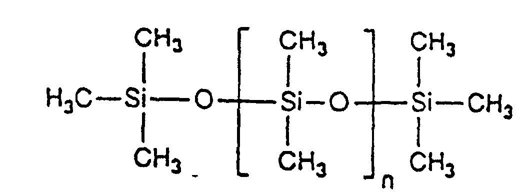 où n est un entier inférieur à 5000,
et les diméthiconols qui sont des diméthylsilicones terminés par des groupes hydroxy.

Comme exemple de polydiméthylsiloxanes modifiés, on citera les diméthicone copolyols qui sont des polymères de diméthylsiloxane comportant des chaînes latérales polyoxyéthylène et/ou polyoxypropylène.

La composition polymère adhésive à base de silicone représente de préférence 5 à 25 % du poids de la composition.

Les promoteurs d'absorption peuvent être choisis notamment parmi le propylèneglycol, l'hexylèneglycol, le dipélargonate de propylène glycol, l'éther monoéthylique de glycéryle, le diéthylène glycol, des monoglycérides, du monooléate de glycérides éthoxylés (avec 8 à 10 motifs oxyde d'éthylène), l'azone (1-dodécyl azacycloheptane-2-one), le 2-(n-nonyl)-1,3-dioxolanne, le myristate d'isopropyle, le myristate d'octyle, le myristate de dodécyle, l'alcool myristylique, l'alcool laurylique, l'acide laurique, le lactate de lauryle, le terpinol, le 1-menthol, le d-limonène, la β-cyclodextrine et ses dérivés ou des agents tensioactifs tels que polysorbates, esters de sorbitan, esters de saccharose, acides gras, sels biliaires, ou encore des produits lipophiles et/ou hydrophiles et/ou amphiphiles tels que esters du polyglycérol, N-méthylpyrrolidone, glycérides polyglycosylés et lactate de cétyle.

Le promoteur d'absorption représente de préférence de 5 à 25 % du poids de la composition.

Comme solvant volatil on utilise essentiellement des silicones ou polysiloxanes volatils et notamment des polydiméthylcyclosiloxanes (ou cyclométhicone), c'est-à-dire des composés de formule : 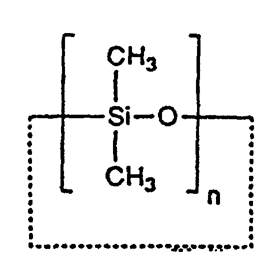 où n est en moyenne entre 3 et 6 et notamment des composés où n = 4 ou 5, ainsi que les polysiloxanes linéaires tels que l'hexaméthyl disiloxane ou des diméthicones de faible masse moléculaire.

Les silicones volatiles représentent de préférence de 50 à 85 % du poids de la composition.

On peut également utiliser en plus jusqu'à 25 % et de préférence jusqu'à 20 % d'autres solvants volatils tels que éthanol, isopropanol, chloroforme, heptane, acétate d'éthyle, à l'exclusion de l'eau. Il est à noter en effet que l'eau n'est pas compatible avec les polysiloxanes utilisés et doit être évitée.

Le gaz propulseur peut être tout gaz propulseur utilisé pour pulvériser une composition liquide présente dans un boitier pour aérosol. Ce peut être notamment un gaz propulseur de type HFC tel que le HFC 134A (CH₂F-CF₃) développé pour remplacer les CFC ou encore des gaz propulseurs relativement intertes tels que l'azote et le gaz carbonique.

On utilisera de préférence des boitiers munis d'une valve doseuse permettant de pulvériser des quantités déterminées de composition sous pression. Cela permet de délivrer une quantité déterminée de principe actif sur la peau.

On peut en outre utiliser une valve dont la sortie est munie d'un cône limitant la dispersion de la pulvérisation. On forme ainsi sur des zones déterminées de la peau des pellicules capables de libérer de façon régulière des quantités déterminées de principe actif.

On donnera ci-après des exemples de compositions selon l'invention.

### Exemple 1 : Composition à base d'acide rétinoïque

On dissout 50 mg d'acide rétinoïque dans 20 g d'isopropanol. On ajoute une solution de 1 g de silicone constituée par une solution à 13 % de diméthiconol dans de la cyclométhicone volatile, préalablement mélangée avec 60 g de polydiméthylcyclosiloxane volatil.

On introduit la solution obtenue dans des boitiers pour aérosol en aluminium, à raison de 10 ml par boitier. On ferme les boitiers par des valves doseuses.

Enfin, on remplit les boitiers avec un agent propulseur (CH₂F-CF₃) sous pression (environ 5 à 7.10⁵ Pa) dans le boitier.

On obtient une composition qui contient dans chaque boitier en poids:

| | |
|---|---|
| Acide rétinoïque | 0,05 % |
| Isopropanol | 20 % |
| Silicone volatile (polydiméthylcyclosiloxane) | 60% |
| Diméthiconol à 13 % dans cyclométhicone | 1% |
| CH₂F-CF₃ | 18,95%. |

Par pression sur la valve doseuse, 100 microlitres de cette composition sont pulvérisés sur la peau sous forme de fines gouttelettes qui se déposent sur la peau en formant une pellicule contenant de l'acide rétinoïque dans une matrice de silicone.

Une dose de 100 microlitres forme une pellicule contenant 50 microgrammes d'acide rétinoïque.

Cette pellicule libère progressivement l'acide rétinoïque à travers la peau.

Une telle composition peut être utilisée pour un traitement de l'acné.

### Exemple 2 : Composition à base de bétaméthasone.

On opère comme à l'exemple 1 pour préparer une composition contenant :

| | |
|---|---|
| Bétaméthasone dipropionate (exprimé en bétaméthasone) | 0,05 % |
| Propylène glycol | 10 % |
| Silicone volatile (polydiméthylcyclosiloxane) | 60 % |
| Diméthiconol à 13 % dans cyclométhicone | 2% |
| CH₂F-CF₃ | 27,95 %. |

Une dose de 100 microlitres forme une pellicule contenant 50 microgrammes de bétaméthasone.

### Exemple 3 : Composition à base de lidocaïne.

On opère comme à l'exemple 1 pour préparer une composition contenant :

| | |
|---|---|
| Lidocaïne | 5 % |
| Stéarate d'éthylèneglycol | 10 % |
| Ethanol | 10 % |
| Silicone volatile (polydiméthylcyclosiloxane) | 50 % |
| Diméthiconol à 13 % dans cyclométhicone | 2 % |
| CH₂F-CF₃ | 23 %. |

Une dose de 100 microlitres forme une pellicule contenant 5 mg de lidocaïne.

### Exemple 4: Composition à base de kétoconazole.

On opère comme à l'exemple 1 pour préparer une composition contenant :

| | |
|---|---|
| Kétoconazole | 2 % |
| Propylène glycol | 10% |
| Polysorbate 60 | 1 % |
| Silicone volatile (polydiméthylcyclosiloxane) | 60% |
| Diméthiconol à 13 % dans cyclométhicone | 1 % |
| CH₂F-CF₃ | 26 %. |

Une dose de 100 microlitres forme une pellicule contenant 2 mg de kétoconazole.

## Revendications

1. Composition destinée à former sur la peau, par pulvérisation à partir d'un boitier pour aérosol, une pellicule pour l'administration transdermique d'un principe actif, qui comprend :
a) un principe actif lipophile
b) de 0,5 à 25 % en poids, et avantageusement de 5 à 25 % en poids, d'une composition polymère adhésive à base de silicone
c) de 0 à 25 % en poids d'un promoteur d'absorption
d) de 25 à 95 % en poids d'un solvant volatil comprenant des silicones volatiles, et
e) de 0,5 à 50 % en poids d'un gaz propulseur sous pression.

2. Composition selon la revendication 1, dans laquelle la composition polymère adhésive comprend des polysiloxanes.

3. Composition selon la revendication 2, dans laquelle les silicones volatiles sont des polydiméthylcyclosiloxanes.

4. Composition selon l'une quelconque des revendications 1 à 3, qui comprend de 50 à 85 % de silicone volatile.

5. Composition selon l'une quelconque des revendications 1 à 4, qui comprend de 0 à 25 % de solvant volatil autre qu'une silicone volatile.

6. Composition selon l'une quelconque des revendications 1 à 4, qui comprend de 0 à 20 % de solvant volatil autre qu'une silicone volatile.

7. Composition selon l'une quelconque des revendications 1 à 6, présente dans un boîtier pour aérosol muni d'une valve doseuse.

8. Boîtier pour aérosol destiné à former sur la peau, par pulvérisation, une pellicule pour l'administration transdermique d'un principe actif, comprenant :
a) un principe actif lipophile
b) de 0,5 à 25% en poids, et avantageusement de 5 à 25% en poids, d'une composition polymère adhésive à base de silicone
c) de 0 à 25% en poids d'un promoteur d'absorption
d) de 25 à 95% en poids d'un solvant volatil comprenant des silicones volatiles, et
e) de 0,5 à 50% en poids d'un gaz propulseur sous pression.

## Patentansprüche

1. Zusammensetzung, die vorgesehen ist, durch Versprühen aus einem Aerosolbehälter auf der Haut einen Film für die transdermale Verabreichung eines Wirkstoffs zu bilden, und welche
a) einen lipophilen Wirkstoff,
b) 0,5 bis 25 Gew.% und vorteilhafterweise 5 bis 25 Gew.% einer haftfähigen Polymerzusammensetzung auf der Basis von Silicon,
c) 0 bis 25 Gew.% eines Absorptionsverstärkers,
d) 25 bis 95 Gew.% eines flüchtige Silicone enthaltenden flüchtigen Lösungsmittels und
e) 0,5 bis 50 Gew.% eines unter Druck stehenden Treibgases
umfasst.

2. Zusammensetzung nach Anspruch 1, in welcher die haftfähige Polymerzusammensetzung Polysiloxane enthält.

3. Zusammensetzung nach Anspruch 2, in welcher die flüchtigen Silicone Polydimethylcyclosiloxane sind.

4. Zusammensetzung nach einem der Ansprüche 1 bis 3, die 50 bis 85 % flüchtiges Silicon enthält.

5. Zusammensetzung nach einem der Ansprüche 1 bis 4, die 0 bis 25 % flüchtiges Lösungsmittel enthält, das kein flüchtiges Silicon ist.

6. Zusammensetzung nach einem der Ansprüche 1 bis 4, die 0 bis 20 % flüchtiges Lösungsmittel enthält, das kein flüchtiges Silicon ist.

7. Zusammensetzung nach einem der Ansprüche 1 bis 6, die in einem mit einem Dosierventil ausgestatteten Aerosolbehälter vorliegt.

8. Behälter für ein Aerosol, das vorgesehen ist, durch Versprühen auf der Haut einen Film zur transdermalen Verabreichung eines Wirkstoffs zu bilden, und welches
a) einen lipophilen Wirkstoff,
b) 0,5 bis 25 Gew.% und vorteilhafterweise 5 bis 25 Gew.% einer haftfähigen Polymerzusammensetzung auf der Basis von Silicon,
c) 0 bis 25 Gew.% eines Absorptionsverstärkers,
d) 25 bis 95 Gew.% eines flüchtige Silicone enthaltenden flüchtigen Lösungsmittels und
e) 0,5 bis 50 Gew.% eines unter Druck stehenden Treibgases
umfasst.

## Claims

1. Composition intended to form on the skin, by spraying from an aerosol can, a film for the transdermal administration of an active principle, this composition comprising:
a) a lipophilic active principle
b) from 0.5 to 25% by weight, and advantageously from 5 to 25% by weight, of a silicone-based adhesive polymer composition
c) from 0 to 25% by weight of an absorption promoter
d) from 25 to 95% by weight of a volatile solvent comprising volatile silicones, and
e) from 0.5 to 50% by weight of a pressurized propellent gas.

2. Composition according to Claim 1, in which the adhesive polymer composition comprises polysiloxanes.

3. Composition according to Claim 2, in which the volatile silicones are polydimethylcyclosiloxanes.

4. Composition according to any one of Claims 1 to 3, which comprises from 50 to 85% of volatile silicone.

5. Composition according to any one of Claims 1 to 4, which comprises from 0 to 25% of volatile solvent other than a volatile silicone.

6. Composition according to any one of Claims 1 to 4, which comprises from 0 to 20% of volatile solvent other than a volatile silicone.

7. Composition according to any one of Claims 1 to 6, which is present in an aerosol can fitted with a metering valve.

8. Can for an aerosol which is intended to form on the skin, by spraying, a film for the transdermal administration of an active principle, this aerosol comprising:
a) a lipophilic active principle
b) from 0.5 to 25% by weight, and advantageously from 5 to 25% by weight, of a silicone-based adhesive polymer composition
c) from 0 to 25% by weight of an absorption promoter
d) from 25 to 95% by weight of a volatile solvent comprising volatile silicones, and
e) from 0.5 to 50% by weight of a pressurized propellent gas.
